# EUROPÄISCHE PATENTANMELDUNG

(11) **EP 0 930 049 A1**
(43) Veröffentlichungstag der Anmeldung: **21.07.1999**
(21) Anmeldenummer: 98122825.7
(22) Anmeldetag: 01.12.1998
(51) Int. Cl.: A61B 17/68

(54) **Knochennagel**

(30) Priorität: 15.01.1998 DE 19801219
(71) Anmelder: Essiger, Holger K., Dr., 30900 Wedemark (DE)
(72) Erfinder: Essiger, Holger K., Dr., 30900 Wedemark (DE)
(74) Vertreter: Depmeyer, Lothar

(57) **Zusammenfassung**

Die Erfindung betrifft einen Knochennagel mit einem sich zum freien Ende verjüngenden Schaft und einem vorzugsweise tellerartigen Kopf. Um diese Nägel besser eintreiben und hiernach besser gegen ein Lösen sichern zu können, wird aufgrund der Erfindung vorgeschlagen, den Schaft zumindest über einen Teil seiner Länge mit einem oder mehreren Vorsprüngen zu versehen, die vorzugsweise kantig, insbesondere spitz auslaufend ausgeführt sind. Vorzugsweise ist der Schaft auch mit längs verlaufenden Leisten ausgestattet.

## Beschreibung

Die Erfindung betrifft einen für die Knochenchirurgie bestimmten Knochennagel, insb. zum Befestigen von Membranen, Folien und Schienen, mit einem Schaft und einem vorzugsweise tellerartigen Kopf.

Bei bekannten Knochennägeln mit im Querschnitt rundem Schaft ergeben sich beim Eintreiben der Nägel Schwierigkeiten, die selbst dann nicht behoben werden können, wenn das Eintreiben durch sog. Vorbohren vorbereitet wird. Abgesehen davon, dass mit dem Vorbohren ein nicht unerheblicher Zeitaufwand verbunden ist, biegen die Schäfte oft zur Seite ab und sind oft auch ausserstande, den Nagel ausreichend sicher zu halten, wenn er sich in seiner Wirkstellung befindet. Diese Nachteile machen sich besonders bei zur Halterung von Folien, Membranen und ähnlichen dünnwandigen Elementen der Chirurgie vorgesehenen Nägeln bemerkbar, die wegen der Dünnwandigkeit der vorerwähnten Elemente eine vergleichsweise kleine, im Millimeterbereich liegende Schaftlänge aufweisen.

Aufgrund der Erfindung sollen diese Nachteile beseitigt, zumindest aber wesentlich vermindert werden. Demgemäss liegt der Erfindung im wesentlichen die Aufgabe zugrunde, die eingangs erwähnten Nägel so zu verbessern, dass sie besser, schneller und einfacher eingetrieben werden können und/oder nach dem Eintreiben ungewollte Lockerungen bzw. ein Lösen der Nagelung weitgehend vermieden werden.

Zur Lösung dieser Aufgabe sind erfindungsgemäss die Schäfte der Nägel zumindest über einen Teil ihrer Länge mit einem oder mehreren Vorsprüngen versehen, die zweckmässigerweise kantig, insb. scharfkantig ausgebildet werden und sich in Schaftlängsrichtung erstrecken, aber auch in Reihen angeordnet werden können, die sich vorzugsweise in Längsrichtung des Schaftes erstrecken.

Diese Vorsprünge ermöglichen überraschenderweise ein vergleichsweise einfaches Eintreiben der Nägel, das sich durch die ggfs. schneidende Wirkung der Vorsprünge erklären lässt. Sind die Vorsprünge in Einzelanordnung vorgesehen, so können sie zusätzlich noch, aber auch für sich kleine Verankerungen im Knochen bilden, um eine Lockerung der Nagelverbindung zu unterbinden. Es ist dabei vorteilhaft, wenn die Vorsprünge ein Eintreiben des Nagels nicht oder nicht wesentlich behindern, jedoch im eingetriebenen Zustand der Nägel widerhakenähnliche Wirkungen ermöglichen, z.B. nach Art von Sägezähnen gestaltet werden.

Es ist andererseits möglich, die Vorsprünge so auszuführen, dass sie sich leistenartig zumindest über einen Teil der Schaftlänge erstrecken. Aus diesen Gründen kann bei der Wahl der Querschnitte der Nagelschäfte bereits auf diese Vorschläge Rücksicht genommen werden, indem die Schäfte in den gewünschten Abschnitten dreieckig, quadratisch, rechteckig, sternförmig oder ähnlich gestaltet werden.

Ein besonders vorteilhafter Vorschlag geht dahin, die Schäfte auf einem Teil ihrer Länge mit leistenartigen, kantigen Vorsprüngen und auf einem anderen Teil ihrer Länge mit einzelnen, ggfs. in Reihen angeordneten, widerhakenähnlichen Vorsprüngen auszustatten, wobei vorzugsweise die Leisten am freien Ende des Schaftes und die Widerhaken zum Kopf des Nagels hin vorgesehen werden. Aufgrund der Gestaltung des Schaftes am freien Ende ergeben sich Vorteile im Hinblick auf das Eintreiben des Nagels, während zum Kopf des Nagels hin die Mittel vorgesehen sind, die ein Lockern oder einen Verlust des Nagels unterbinden.

Weitere Einzelheiten der Erfindung werden anhand der Zeichnung erläutert, in der Ausführungsbeispiele der Erfindung dargestellt sind. Es zeigen :
Fig. 1 einen in Wirkstellung befindlichen, eingetriebenen Knochennagel zur Befestigung einer Folie oder einer Membran im Schnitt,
Fig. 2 einen Schnitt nach der Linie II - II von Fig. 1,
Fig. 3 eine Nagelteilansicht in Richtung des Pfeiles III von Fig. 2,
Fig. 4, 5 und 6 je einen gegenüber Fig. 2 abgewandelten Schaftquerschnitt,
Fig. 7 einen Schnitt nach der Linie VII - VII von Fig. 1 und
Fig. 8 einen Teillängsschnitt durch den Nagelschaft gemäss Fig. 1 im Bereich eines widerhakenähnlichen Vorsprunges auf dem Nagelschaft nach der Linie VIII - VIII von Fig. 7.

Der aus Stahl od. dgl., insb. Titan, bestehende Knochennagel hat einen tellerartigen Kopf 1 und einen schlanken Schaft 2, der die zu befestigende Membran 3 durchsetzt und in den Knochen 4 eingreift.

Der Schaft 2 hat zwei im wesentlichen gleich lange Abschnitte 5, 6, von denen der Abschnitt 5 in besonderer Weise so ausgeführt ist, dass er das Eintreiben des Nagels - vorzugsweise ohne Vorbohren - erleichtert. Der Abschnitt 6 hingegen ist vorzugsweise so ausgebildet, dass er in der Lage ist, eine Lockerung oder gar ein ungewolltes Entfernen des Nagels zu verhindern.

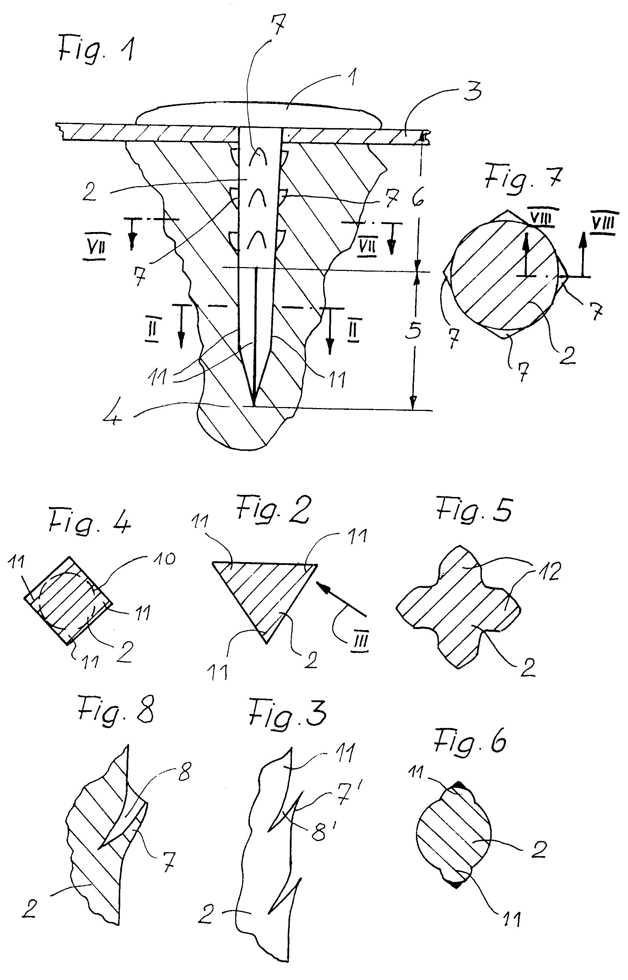

Der Abschnitt 6 kennzeichnet sich durch über den Umfang des Schaftes 2 verteilt angeordnete, senkrechte Reihen aus Vorsprüngen 7, die widerhakenartig ausgeführt sind und deren spitz auslaufendes Ende zum Kopf 1 hin zeigt. Diese Vorsprünge 7 sind nach Art von sog. Feilenhieben für grobe Feilen ( Holzfeilen ) ausgeführt und durch Herausbiegen aus dem Material des Schaftes 2 gebildet. Die Vorsprünge 7 sind nicht nur gemäss Fig. 8 im Schnitt und in der Seitenansicht zum freien Ende hin spitz auslaufend , sondern auch ähnlich zum freien Ende sich verjüngend ausgeführt, wenn man sie gemäss Fig. 1 in der Ansicht betrachtet. Dadurch ergibt sich eine besonders gute Fixierung der Nägel. Im übrigen sind die Vertiefungen, die die herausgebogenen Vorsprünge 7 hinterlassen mit 8 bezeichnet.

Der Abschnitt 5, der das Eintreiben des Schaftes 2 erleichtern soll, weist ebenfalls Vorsprünge - gegenüber dem bekannten runden Schaftquerschnitt ( vgl. die gestrichelte Linie 10 gemäss Fig. 4 ) - auf. Die Vorsprünge sind jedoch hier längs zum Schaft 2 verlaufende Leisten 11 mit etwa dreieckigem Querschnitt. Diese können in einfacher Weise gebildet werden durch einen dreieckigen Schaftquerschnitt gemäss Fig. 2, aber ggfs. auch durch den quadratischen Querschnitt gemäss Fig. 4.

Es hat sich gezeigt, dass überraschenderweise die kantigen, bzw. spitz auslaufenden Vorsprünge bzw. Leisten 11 beim Eintreiben des Nagels gewissermassen einen Schneideffekt und so eine Art Verdrängerwirkung auf den Knochen 4 ausüben.

Bei bestimmten Anwendungsfällen kann gemäss Fig. 5 auch eine im wesentlichen kreuzförmige Querschnittsgestalt des Schaftes 2 gewählt werden, wobei die das Kreuz bildenden Balken 12 jeweils spitz auslaufen.

Ferner ist es möglich, lediglich zwei - diametral gegenüberliegende - Vorsprünge 11 gemäss Fig. 6 zu wählen.

Sofern bei den Knochennägeln ohnehin ungewollte Lockerungen od. dgl. nicht eintreten können, kann auf die widerhakenähnlichen Vorsprünge verzichtet werden. Die Vorsprünge 11 können dann als durchlaufende Längsleisten auf dem Schaft 2 angebracht werden.

Werden zusätzliche Sicherungen gewünscht, so können zusätzlich zu durchlaufenden Längsleisten einzelne widerhakenähnliche Vosprünge 7 gewählt werden ( wie in Fig. 1 dargestellt ). Dabei ist es zweckmässig, im unteren Teil des Schaftes ( Abschnitt 5 ) die das Eintreiben der Nägel erleichternden Mittel vorzusehen.

Aus Fig. 3 ist ferner erkennbar, dass auch leistenförmige Vorsprünge 11 wiederum stellenweise als Widerhaken oder in ähnlicher Weise ausgeführt sein können. Hier sind die Vorsprünge mit 7' und die hinter ihnen liegenden Vertiefungen mit 8' bezeichnet.

Es sei erwähnt, dass der erfindungsgemässe Knochennagel sinngemäss auch zur Befestigung von Schienen verwendbar ist, wobei der Knochennagel meist eine Ausnehmung der Schiene durchsetzt, um die Halterung herbeizuführen.

## Patentansprüche

1. Knochennagel, insb. zur Befestigung von Membranen, Folien, Schienen und dgl., mit einem sich zum freien Ende verjüngenden Schaft und einem vorzugsweise tellerartigen Kopf, dadurch gekennzeichnet, dass der Schaft (2) zumindest über einen Teil seiner Länge mit einem oder mehreren Vorsprüngen (7,11) versehen ist.

2. Nagel nach Anspruch 1, dadurch gekennzeichnet, dass die Vorsprünge (11,7) am freien Ende nach aussen hin kantig, vorzugsweise spitz auslaufend ausgebildet sind.

3. Nagel nach Anspruch 1, dadurch gekennzeichnet, dass die Vorsprünge sich in Schaftrichtung erstreckende Leisten (11) sind.

4. Nagel nach Anspruch 1, dadurch gekennzeichnet, dass die Vorsprünge (7) in Einzelanordnung bzw. in Schaftlängrichtung gesehen hintereinander vorgesehen sind.

5. Nagel nach Anspruch 1, dadurch gekennzeichnet, dass die Vorsprünge (7) widerhakenartig ausgebildet sind und in Eintreibrichtung des Nagels im Vergleich zur entgegengesetzten Richtung einen kleineren Bewegungswiderstand bieten.

6. Nagel nach Anspruch 1, dadurch gekennzeichnet, dass sich über einen Teil der Schaftlänge durchgehende, leistenförmige Vorsprünge (11) und über einen anderen Teil der Schaftlänge Vorsprünge (7) mit Widerhakeneffekt erstrecken.

7. Nagel nach Anspruch 6, dadurch gekennzeichnet, dass sich die leistenförmigen Vorsprünge (11) am freien Ende des Schaftes (2) befinden.

8. Nagel nach Anspruch 5, dadurch gekennzeichnet, dass die widerhakenartigen Vorsprünge (7) durch Herausbiegen aus dem Material des Schaftes (2) gebildet sind ( Fig. 8 ).

9. Nagel nach Anspruch 5, dadurch gekennzeichnet, dass die widerhakenartigen Vorsprünge (7) sowohl im Längsschnitt des Schaftes als auch in der Ansicht des Schaftes betrachtet sich zum freien Ende hin verjüngen.

10. Nagel nach Anspruch 1, dadurch gekennzeichnet, dass der Schaft (2) zumindest über einen Teil seiner Länge dreieckig oder viereckig, insb. etwa quadratisch, gestaltet ist.

11. Nagel nach Anspruch 1, dadurch gekennzeichnet, dass der Schaft (2) zumindest über einen Teil seiner Länge im wesentlichen kreuzförmig gestaltet ist ( Fig. 5 ).

12. Nagel nach Anspruch 1, dadurch gekennzeichnet, dass sich die Vorsprünge (11) nur an diametral gegenüberliegenden Stellen des Schaftes (2) befinden (Fig. 6).

13. Nagel nach den Ansprüchen 1, 2, 5 und 8, dadurch gekennzeichnet, dass die Widerhaken an den Kanten der leistenförmigen Vorsprünge (11) vorgesehen sind (Fig. 3).
